(19) Europäisches Patentamt
European Patent Office
Office européen des brevets

(11) Numéro de publication : **0 449 717 B1**

(12) **FASCICULE DE BREVET EUROPEEN**

(45) Date de publication du fascicule du brevet : **15.06.94 Bulletin 94/24**

(51) Int. Cl.$^5$ : **A23L 1/221,** A23L 1/064, A61K 7/48

(21) Numéro de dépôt : **91400802.4**

(22) Date de dépôt : **26.03.91**

# (54) Compositions à base de plantes fraîches ou sèches et leurs applications.

(30) Priorité : **30.03.90 FR 9004091**

(43) Date de publication de la demande : **02.10.91 Bulletin 91/40**

(45) Mention de la délivrance du brevet : **15.06.94 Bulletin 94/24**

(84) Etats contractants désignés : **AT BE DE ES FR GB IT NL**

(56) Documents cités :
**EP-A- 0 234 143**
**FR-A- 1 553 029**
**FR-A- 2 201 043**
**FR-A- 2 417 266**

(73) Titulaire : **LABORATOIRES ARDEVAL**
**29 rue Marcel Cachin**
**F-94204 Ivry sur Seine (FR)**

(72) Inventeur : **Derrieu, Guy**
**Le Riviera Parc,**
**33 Chemin du Lautin**
**F-06800 Cagnes Sur Mer (FR)**

(74) Mandataire : **Orès, Bernard et al**
**Cabinet ORES**
**6, Avenue de Messine**
**F-75008 Paris (FR)**

EP 0 449 717 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à des compositions à base de plantes fraîches ou sèches ainsi qu'à leurs applications thérapeutique, cosmétologique et alimentaire.

On entend, au sens de la présente invention, par plante, aussi bien la plante entière que des parties de plante telles que racines, bulbes, tubercules, tiges, feuilles, sommités fleuries, fleurs, fruits ou graines.

Il est connu que les plantes constituent de véritables réservoirs de principes actifs et sont toujours très utilisées soit telles quelles, sous forme sèche notamment, soit sous forme d'extraits, soit sous de nombreuses autres formes dans de nombreuses préparations à usage pharmaceutique, vétérinaire, cosmétologique ou alimentaire.

La conservation des plantes fraîches n'est pas aisée. Dans le Brevet européen 85 589, un procédé de préparation de cellules fraîches stabilisées, qui préserve la quasi totalité des principes actifs de la cellule, est décrit. Ce procédé comporte notamment un broyage à froid et des micronisations effectuées en présence d'un solvant miscible à l'eau et à froid. Néanmoins pour garantir la stabilité dans une préparation hydroorganique, la quantité de plante mise en jeu ne peut être que faible, voisine de 5%, et la quantité de solvant organique élevée, voisine de 30% (m/m).

Dans la demande de Brevet européen 234 143, un procédé de préparation de crèmes, pommades, ou pâtes à base de plantes fraîches a été décrit. Toutefois, bien que présentant une stabililisation améliorée de la plante fraîche par rapport à l'art antérieur, cette demande de Brevet ne permet pas d'obtenir une stabilisation totale de tous les constituants de la plante fraîche. En effet la plante fraîche congelée, cryobroyée puis micronisée est stabilisée par l'adjonction d'un gélifiant naturel ou synthétique, c'est-à-dire que les particules végétales sont enrobées, enveloppées par le gélifiant. L'apport de ce constituant va protéger la particule végétale d'une agression extérieure, mais ne bloque pas toutes les réactions enzymatiques intracellulaires.

La présente invention s'est en conséquence donné pour but de pourvoir à de nouvelles compositions pâteuses stabilisées à base de plantes fraîches qui répondent mieux aux nécessités de la pratique que les formes antérieurement connues, notamment en ce qu'elles permettent d'obtenir la stabilisation et l'immobilisation de tous les éléments natifs de la (des) plante(s) et ceci même pour des compositions contenant plus de 50 % (m/m ou m/v) de plante.

La présente invention a pour objet une composition pâteuse stabilisée à base de plante(s) fraîche(s) et/ou sèche(s), sous la forme de particules d'une granulométrie de quelques micromètres à quelques millimètres, obtenues après nettoyage poussé desdites plantes, par cryobroyage à une température inférieure à 0°C, laquelle composition est caractérisée en ce qu'elle comprend :

- au moins 50 % desdites particules de plantes ;
- au moins un solvant ayant la propriété de bloquer les réactions de dégradation et dont la quantité minimum dépend de sa nature et varie de 8 à moins de 45 % ; et
- au moins 10 % d'un agent absorbant et séquestrant.

Conformément à l'invention, ladite composition contient en outre divers autres constituants notamment choisis dans le groupe qui comprend les corps gras, les anti-oxydants, les correcteurs de pH, les émulgateurs et les gélifiants naturels ou synthétiques.

Conformément à l'invention le solvant est avantageusement choisi dans le groupe qui comprend le alcools, et plus particulièrement l'éthanol, le propylène glycol ou le glycérol.

Le solvant est choisi de manière à être compatible avec les autres ingrédients de la composition et sa quantité minimum dépend de sa nature et est notamment de 8 % pour l'éthanol et de 40 % pour le glycérol.

Conformément à l'invention, l'agent absorbant et séquestrant est avantageusement choisi dans le groupe qui comprend les maltodextrines, les cyclodextrines, les sucres, les silices, les diatomées, les zéolithes.

Les plantes sont de préférence des plantes à usage médicinal, cosmétique (dermatologique ou capillaire), aromatique, tinctorial ou alimentaire.

Les compositions conformes à l'invention trouvent application dans le domaine pharmaceutique, cosmétologique ou alimentaire.

La composition conforme à l'invention est avantageusement préparée comme suit :

- la plante fraîchement récoltée est fragilisée par congélation brutale à une température inférieure à 0°C et de préférence comprise entre -80°C et -120°C ;
- ladite plante congelée est soumise à au moins un broyage cryogénique jusqu'à l'obtention d'une granulométrie comprise entre quelques micromètres et plusieurs millimètres ;
- on malaxe lentement au moins 50 % (en v/v ou en m/m) de cryobroyat de plante congelée avec au moins 8 % (v/v) de solvant et au moins 10 % (m/m) d'agent absorbant et séquestrant dans un mélangeur ; et
- sous agitation lente, on laisse la température revenir à la température ambiante.

Outre les disposition qui précèdent, l'invention comprend encore d'autres dispositions, qui ressortiront de

la description qui va suivre, qui se réfère à des exemples de compositions objet de la présente invention.

**EXEMPLE 1 : Préparation à base d'ail**

Formule:

| | |
|---|---|
| Ail cryobroyé (morceaux de 2 mm) ...... | 60 kg |
| Ethanol ............................... | 15 kg |
| Maltodextrines ........................ | 15 kg |
| Lecithine de soja ..................... | 4 kg |
| Huile d'arachide ...................... | 5,9 kg |
| Metabisulfite de sodium ............... | 0,1 kg |

On prépare un cryobroyat de bulbes comme décrit dans la demande de Brevet européen n° 85 589 : les bulbes, frais ou secs (s'ils n'ont pas été blessés), sont totalement congelés à un minimum de -80° C par de l'azote liquide. Après un broyage, la taille des particules selon la convenance peut être de quelques millimètres à quelques dizaines de micromètres.

Le végétal cryobroyé est malaxé lentement avec l'éthanol, les maltodextrines et le métabisulfite de sodium. On laisse revenir, sous agitation lente, à la température ambiante.

On achève la préparation en introduisant les deux autres composés.

Les principes actifs intéressants de l'ail sont l'alliine et l'allicine, mais lorsque l'alliine est en présence d'alliinase, la réaction suivante se produit :

```
                 enzyme
Alliine  ------------>  Allicine  ----->  produits
               alliinase    (instable)        de dégradation
```

Cette dégradation se produit notamment lorsque les bulbes sont blessés ou quand on hache ou broie de l'ail à température ambiante.

L'étude de la stabilité de la composition conforme à l'invention montre qu'il y a blocage de la réaction enzymatique et stabilisation de toute l'alliine.

**EXEMPLE 2 : Préparation à base de basilic**

Formule :

| | | |
|---|---|---|
| Basilic cryobroyé (morceau de 4 mm) ... | | 60 kg |
| Ethanol ............................... | | 10 kg |
| Maltodextrines ........................ | | 20 kg |
| Lecithine de soja ..................... | | 4 kg |
| Huile d'arachide ...................... | | 5,4 kg |
| Metabisulfite de sodium ............... | | 0,1 kg |
| Gomme xanthane ........................ | | 0,5 kg |
| Jus de citron ................... | qsp | pH 5 |

On prépare selon le protocole décrit dans le premier exemple une pâte alimentaire de basilic. La gomme xanthane est introduite et le pH est ajusté à 5 avec du jus de citron, en fin de manipulation.

On note une parfaite stabilité du basilic, dans la mesure où tous les principes odorants fragiles et volatiles sont conservés pendant au moins 18 mois comme l'ont montré des études chromatographiques en phase gazeuse.

**EXEMPLE 3 : Préparation à base de Reine des prés**

Formule :

| | |
|---|---|
| Tige stérile de Reine des prés microcryobroyée .. | 50 kg |
| Ethanol .................................... | 20 kg |
| Propylène glycol ............................ | 7 kg |
| Cyclodextrines .............................. | 10 kg |
| Silice précipitée ........................... | 2 kg |
| Silicones ................................... | 2 kg |
| Huile de vaseline ........................... | 5 kg |
| Emulgateur .................................. | 3 kg |
| Gomme guar .................................. | 1 kg |

Préparée selon le protocole décrit dans l'exemple 1, cette composition sous forme d'onguent se révèle particulièrement efficace contre les douleurs rhumatismales et les inflammations des articulations.

Ces activités sont dues à la présence des dérivés salicylés sous forme d'hétérosides stabilisés.

**EXEMPLE 4 : Dégradation : essai comparatif entre une composition conforme à l'invention, une composition comprenant une plante et un solvant, une composition comprenant une plante et un agent absorbant et séquestrant.**

| | PRODUIT DE DEGRADATION |
|---|---|
| COMPOSITION DE L'EXEMPLE 1 | 0 % |
| Ail + alcool (15 %) | 20 % |
| Ail + maltodextrine (15 %) | 85 % |

Ce Tableau montre que, de manière inattendue, une composition conforme à l'invention inhibe la dégradation des principes actifs de la plante qu'elle contient alors que des compositions ne contenant qu'un solvant ou qu'un agent absorbant et séquestrant, associé à la plante n'inhibe pas la dégradation des principes actifs de cette dernière.

**EXEMPLE 5 : Dégradation : rôle de la concentration en solvant.**

| | Composition 1 selon l'exemple 1 | Composition 2 | Composition 3 | Composition 4 |
|---|---|---|---|---|
| Ail cryobroyé | 60 % | 60 % | 60 % | 55 % |
| Ethanol | 15 % | 0 | 8 % | 0 |
| Glycérol | 0 | 15 % | 0 | 35 % |
| Maltodextrines | 15 % | 15 % | 22 % | 0 |
| Autres constituants | 10 % | 10 % | 10 % | 10 % |
| Produits de dégradation | 0 % | 35 % | < 1 % | < 5 % |

Les compositions 2, 3 et 4 sont des variantes de la composition 1 selon l'exemple 1.

Le Tableau ci-dessus montre une dégradation importante dans la composition 2 qui ne comprend pas d'éthanol et une quantité en glycérol inférieure à 40 % ; de plus, si l'on compare les compositions 2 et 4, on voit bien qu'en augmentant la quantité en glycérol on obtient une stabilisation de la composition.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

**Revendications pour les Etats contractants suivants : AT, BE, DE, FR, GB, IT, NL**

**1.** Composition pâteuse stabilisée à base de plante(s) fraîche(s) et/ou sèche(s), sous la forme de particules d'une granulométrie de quelques micromètres à quelques millimètres, obtenues après nettoyage poussé desdites plantes, par cryobroyage à une température inférieure à 0°C, laquelle composition est caractérisée en ce qu'elle comprend :
- au moins 50 % desdites particules de plantes ;
- au moins un solvant ayant la propriété de bloquer les réactions de dégradation et dont la quantité minimum dépend de sa nature et varie de 8 à moins de 45 % ; et
- au moins 10 % d'un agent absorbant et séquestrant.

**2.** Composition selon la revendication 1, caractérisée en ce que le solvant est choisi dans le groupe des alcools.

**3.** Composition selon la revendication 2, caractérisée en ce que l'alcool est sélectionné dans le groupe qui comprend notamment l'éthanol et les glycols.

**4.** Composition selon l'une quelconque des revendications 1 à 3, caractérisée en ce qu'elle comprend au moins 8 % d'éthanol et/ou au moins 40 % de glycérol.

**5.** Composition selon l'une quelconque des revendications 1 à 4, caractérisée en ce que l'agent absorbant et séquestrant est choisi dans le groupe qui comprend les maltodextrines, les cyclodextrines, les sucres,

les silices, les diatomées, les zéolithes.

6. Application de la composition selon l'une quelconque des revendications 1 à 5, à la préparation de médicament.

7. Application de la composition selon l'une quelconque des revendications 1 à 5, à la cosmétologie.

8. Application de la composition selon l'une quelconque des revendications 1 à 5, à la préparation de produit alimentaire.

**Revendications pour l'Etat contractant suivant : ES**

1. Procédé de préparation d'une composition pâteuse stabilisée à base de plante(s) fraîche(s) et/ou sèche(s), sous la forme de particules d'une granulométrie de quelques micromètres à quelques millimètres, obtenues après nettoyage poussé desdites plantes, par cryobroyage à une température inférieure à 0°C, laquelle composition comprend :
   - au moins 50 % desdites particules de plantes ;
   - au moins un solvant ayant la propriété de bloquer les réactions de dégradation et dont la quantité minimum dépend de sa nature et varie de 8 à moins de 45 % ; et
   - au moins 10 % d'un agent absorbant et séquestrant,
     lequel procédé est caractérisé en ce qu'il comprend :
   - la récolte de la plante qui est fragilisée par congélation brutale à une température inférieure à 0°C et de préférence comprise entre -80°C et -120°C ;
   - le broyage cryogénique de ladite plante congelée jusqu'à l'obtention d'une granulométrie comprise entre quelques micromètres et plusieurs millimètres ;
   - le malaxage lent d'au moins 50 % (en v/v ou en m/m) de cryobroyat de plante congelée avec au moins 8 % (v/v) de solvant et au moins 10 % (m/m) d'agent absorbant et séquestrant dans un mélangeur ; et
   - la remise à la température ambiante sous agitation lente.

2. Procédé selon la revendication 1, caractérisé en ce que le solvant est choisi dans le groupe des alcools.

3. Procédé selon la revendication 2, caractérisée en ce que l'alcool est sélectionné dans le groupe qui comprend notamment l'éthanol et les glycols.

4. Procédé selon l'une quelconque des revendications 1 à 3, caractérisé en ce que ladite composition comprend au moins 8 % d'éthanol et/ou au moins 40 % de glycérol.

5. Procédé selon l'une quelconque des revendications 1 à 4, caractérisé en ce que l'agent absorbant et séquestrant est choisi dans le groupe qui comprend les maltodextrines, les cyclodextrines, les sucres, les silices, les diatomées, les zéolithes.

6. Application de la composition selon l'une quelconque des revendications 1 à 5, à la préparation de médicament.

7. Application de la composition selon l'une quelconque des revendications 1 à 5, à la cosmétologie.

8. Application de la composition selon l'une quelconque des revendications 1 à 5, à la préparation de produit alimentaire.

**Patentansprüche**

**Patentansprüche für folgende Vertragsstaaten : AT, BE, DE, FR, GB, IT, NL**

1. Stabilisierte pastenartige Masse auf der Basis von einer oder mehreren frischen und/oder trockenen Planzen in Form von Teilchen mit einer Teilchengröße von einigen Mikrometern bis einigen Millimetern, die nach gründlicher Reinigung der Pflanzen unter Kaltzerkleinerung bei einer Temperatur unterhalb von 0°C erhalten werden, wobei die Masse dadurch gekennzeichnet ist, daß sie folgende Bestandteile umfaßt:

- mindestens 50% der Pflanzenteilchen,
- mindestens ein Lösungsmittel, das die Eigenschaft hat, Zersetzungsreaktionen zu blockieren, und dessen Mindestmenge von seiner Beschaffenheit abhängt und im Bereich von 8 bis mindestens 45% liegt, und
- mindestens 10% eines Absorptions- und Sequestriermittels.

2. Masse nach Anspruch 1, dadurch gekennzeichnet, daß das Lösungsmittel aus der Gruppe der Alkohole ausgewählt ist.

3. Masse nach Anspruch 2, dadurch gekennzeichnet, daß der Alkohol aus der Gruppe ausgewählt ist, die insbesondere Ethanol und Glykole umfaßt.

4. Masse nach einem der Ansprüche 1 bis 3, dadurch gekennzeichnet, daß sie mindestens 8% Ethanol und/oder mindestens 40% Glycerin umfaßt.

5. Masse nach einem der Ansprüche 1 bis 4, dadurch gekennzeichnet, daß das Absorptions- und Sequestriermittel aus der Gruppe ausgewählt ist, die Maltodextrine, Cyclodextrine, Zucker, Siliziumdioxide, Kieselgure und Zeolithe umfaßt.

6. Verwendung der Masse nach einem der Ansprüche 1 bis 5 zur Herstellung eines Arzneimittels.

7. Verwendung der Masse nach einem der Ansprüche 1 bis 5 in der Kosmetik.

8. Verwendung der Masse nach einem der Ansprüche 1 bis 5 zur Herstellung eines Nahrungsmittels.

**Patentansprüche für folgenden Vertragsstaat : ES**

1. Verfahren zur Herstellung einer stabilisierten pastenartigen Masse auf der Basis von einer oder mehreren frischen und/oder trockenen Planzen in Form von Teilchen mit einer Teilchengröße von einigen Mikrometern bis einigen Millimetern, die nach gründlicher Reinigung der Pflanzen unter Kaltzerkleinerung bei einer Temperatur unterhalb von 0°C erhalten werden, wobei die Masse folgende Bestandteile umfaßt:
   - mindestens 50% der Pflanzenteilchen,
   - mindestens ein Lösungsmittel, das die Eigenschaft hat, Zersetzungsreaktionen zu blockieren, und dessen Mindestmenge von seiner Beschaffenheit abhängt und im Bereich von 8 bis mindestens 45% liegt, und
   - mindestens 10% eines Absorptions- und Sequestriermittels,

   wobei das Verfahren dadurch gekennzeichnet ist, das es die folgende Schritte umfaßt:
   - Abernten der Pflanze, die durch Gefrierschockbehandlung bei einer Temperatur unterhalb von 0°C und vorzugsweise zwischen -80°C und -120°C versprödet wird,
   - Kaltzerkleinerung der tiefgefrorenen Pflanze, bis eine Teilchengröße zwischen einigen Mikrometern und mehreren Millimetern erhalten wird,
   - langsames Durchkneten von mindestens 50% (in Vol./Vol. oder Masse/Masse) der kaltzerkleinerten, tiefgefrorenen Pflanze mit mindestens 8% (Vol./Vol.) Lösungsmittel und mindestens 10% (Masse/Masse) Absorptions- und Sequestriermittel in einer Mischvorrichtung, und
   - Zurückführen der Masse auf Umgebungstemperatur unter langsamem Rühren.

2. Verfahren nach Anspruch 1, dadurch gekennzeichnet, daß das Lösungsmittel aus der Gruppe der Alkohole ausgewählt ist.

3. Verfahren nach Anspruch 2, dadurch gekennzeichnet, daß der Alkohol aus der Gruppe ausgewählt ist, die insbesondere Ethanol und Glykole umfaßt.

4. Verfahren nach einem der Ansprüche 1 bis 3, dadurch gekennzeichnet, daß die Masse mindestens 8% Ethanol und/oder mindestens 40% Glycerin umfaßt.

5. Verfahren nach einem der Ansprüche 1 bis 4, dadurch gekennzeichnet, daß das Absorptions- und Sequestriermittel aus der Gruppe ausgewählt ist, die Maltodextrine, Cyclodextrine, Zucker, Siliziumdioxide, Kieselgure und Zeolithe umfaßt.

6. Verwendung der Masse nach einem der Ansprüche 1 bis 5 zur Herstellung eines Arzneimittels.

7. Verwendung der Masse nach einem der Ansprüche 1 bis 5 in der Kosmetik.

8. Verwendung der Masse nach einem der Ansprüche 1 bis 5 zur Herstellung eines Nahrungsmittels.

## Claims

### Claims for the following Contracting States : AT, BE, DE, FR, GB, IT, NL

1. Stabilized pasty composition based on fresh and/or dried plant(s), in the form of particles having a particle size from a few micrometres to a few millimetres, obtained after forced cleaning of the said plants, by low-temperature crushing at a temperature lower than 0°C, which composition is characterized in that it comprises:
   - at least 50 % of the said plant particles;
   - at least one solvent having a blocking property for degradation reactions and for which the minimum quantity depends on its nature and varies from 8 to less than 45 %; and
   - at least 10 % of an absorbing and sequestering agent.

2. Composition according to Claim 1, characterized in that the solvent is chosen from the alcohol group.

3. Composition according to Claim 2, characterized in that the alcohol is selected from the group which in particular comprises ethanol and glycols.

4. Composition according to any one of Claims 1 to 3, characterized in that it comprises at least 8 % of ethanol and/or at least 40 % of glycerol.

5. Composition according to any one of Claims 1 to 4, characterized in that the absorbing and sequestering agent is chosen from the group which comprises maltodextrins, cyclodextrins, sugars, silicas, diatoms and zeolites.

6. Application of the composition according to any one of Claims 1 to 5 to the preparation of a medicament.

7. Application of the composition according to any one of Claims 1 to 5 to cosmetology.

8. Application of the composition according to any one of Claims 1 to 5 to the preparation of a food product.

### Claims for the following Contracting State : ES

1. Process for the preparation of a stabilized pasty composition based on fresh and/or dried plant(s), in the form of particles having a particle size from a few micrometres to a few millimetres, obtained after forced cleaning of the said plants, by low-temperature crushing at a temperature lower than 0°C, which composition comprises:
   - at least 50 % of the said plant particles;
   - at least one solvent having a blocking property for degradation reactions and for which the minimum quantity depends on its nature and varies from 8 to less than 45 %; and
   - at least 10 % of an absorbing and sequestering agent,
   
   which process is characterized in that it comprises:
   - harvesting of the plant which is embrittled with by sudden freezing at a temperature lower than 0°C, and preferably between -80°C and -120°C;
   - low-temperature crushing of the said frozen plant until a particle size of between a few micrometres and several millimetres is obtained;
   - slow blending of at least 50 % (in v/v or in m/m) of the frozen plant material which has been crushed at low temperature with at least 8 % (v/v) of solvent and at least 10 % (m/m) of absorbing an sequestering agent in a mixer; and
   - returning to room temperature with slow stirring.

2. Process according to Claim 1, characterized in that the solvent is chosen from the alcohol group.

3. Process according to Claim 2, characterized in that the alcohol is selected from the group which in particular comprises ethanol and glycols.

4. Process according to any one of Claims 1 to 3, characterized in that the said composition comprises at least 8 % of ethanol and/or at least 40 % of glycerol.

5. Process according to any one of Claims 1 to 4, characterized in that the absorbing and sequestering agent is chosen from the group which comprises maltodextrins, cyclodextrins, sugars, silicas, diatoms and zeolites.

6. Application of the composition according to any one of Claims 1 to 5 to the preparation of a medicament.

7. Application of the composition according to any one of Claims 1 to 5 to cosmetology.

8. Application of the composition according to any one of Claims 1 to 5 to the preparation of a food product.